# EUROPEAN PATENT APPLICATION

(11) **EP 2 692 300 A1**
(43) Date of publication of application: **05.02.2014**
(21) Application number: 13382303.9
(22) Date of filing: 25.07.2013
(51) Int. Cl.: A61B 17/29, A61B 17/295, A61B 17/17

(54) **Shoulder arthroscopy forceps**

(30) Priority: 31.07.2012 ES 201230836 U
(71) Applicant: Nunez Blanco, Javier, 33011 Oviedo - Asturias (ES)
(72) Inventor: Nunez Blanco, Javier, 33011 Oviedo - Asturias (ES)
(74) Representative: Carvajal y Urquijo, Isabel

(57) **Abstract**

Shoulder arthroscopy forceps comprising an elbow jointed arm 1 and a straight arm 2, articulated together and one of their ends ending in operational hoops, 8 and 9. The elbow jointed arm 1 supports the jaws, 6 and 7 of the forceps at one of its ends, as well as an outgoing 12, located below and behind the fixed jaw 6. The hoop 8 of the elbow jointed arm is crossed by a drilled through hole, 10.

## Description

### Field of the invention

The present invention refers to shoulder arthroscopy forceps, formed by an elbow jointed arm and a straight arm, the latter being articulated in the elbow joint of the elbow jointed arm, this elbow jointed arm being a supporting arm, at the end of one of its stretches, for the two jaws of the forceps, one fixed jaw formed at the end of said stretch and the other articulated to the same, whilst the ends of the other stretch of this elbow jointed arm and the straight arm both finish in operational hoops or rings.

The forceps, object of the present invention, are especially applicable to fractured cuffs of shoulder rotators in any age range. It is important to bear in mind that shoulder pathology affects between 3% and 7% of the general population and that 60% of those affected are affected as a result of rotator cuff disease, a trend which increases with age.

### Background of the invention

Current treatments for the problems set out above entail carrying out an arthroscopy of the shoulder and suture of the torn tendon, by means of fitting moorings and subsequently passing the threads threaded in the mooring through the tendon, using traditional instruments.

### Description of the invention

The object of the present invention is a pair of forceps formed in such a way that a shoulder arthroscopy and suture of the torn tendon can be carried out using technology which is more beneficial than the technology used in traditional systems.

The forceps, object of the present invention, are of the initially indicated variety, formed by an elbow jointed arm and a straight arm which is articulated in the elbow joint of the elbow jointed arm.

In accordance with the invention, the elbow jointed arm has a pointed outgoing by way of a keel on the supporting stretch of the fixed jaw of the forceps, behind and below said jaw, said pointed outgoing being forwards inclining. The operational ring of this elbow jointed arm is crossed by a drilled through hole, located on the same plane and defined by said elbow jointed arm and runs in an inclined position, with a trajectory which cuts the supporting arm of the forceps jaws immediately behind the end of the fixed jaw.

### Brief description of the drawings

The drawings attached represent forceps formed in accordance with the present invention. They provide a non-limiting example thereof, alongside the remaining elements or instruments known to be used in the embodiment of a shoulder arthroscopy. The drawings are as follows:
Figure 1 is a side elevation of forceps formed in accordance with the present invention.
Figure 2 is an upper plan view of the fixed jaw, which forms part of the elbow jointed arm.
Figure 3 is a lower plan view of the same fixed jaw, according to direction A represented in figure 1.
Figure 4 represents the various elements or instruments used in the embodiment of a shoulder arthroscopy.
Figure 5 provides a similar view to that shown in figure 1, representing an alternative embodiment.
Figure 6 is a partial cross section of the forceps, according to the cut line VI-VI shown in figure 5.

### Detailed description of one embodiment

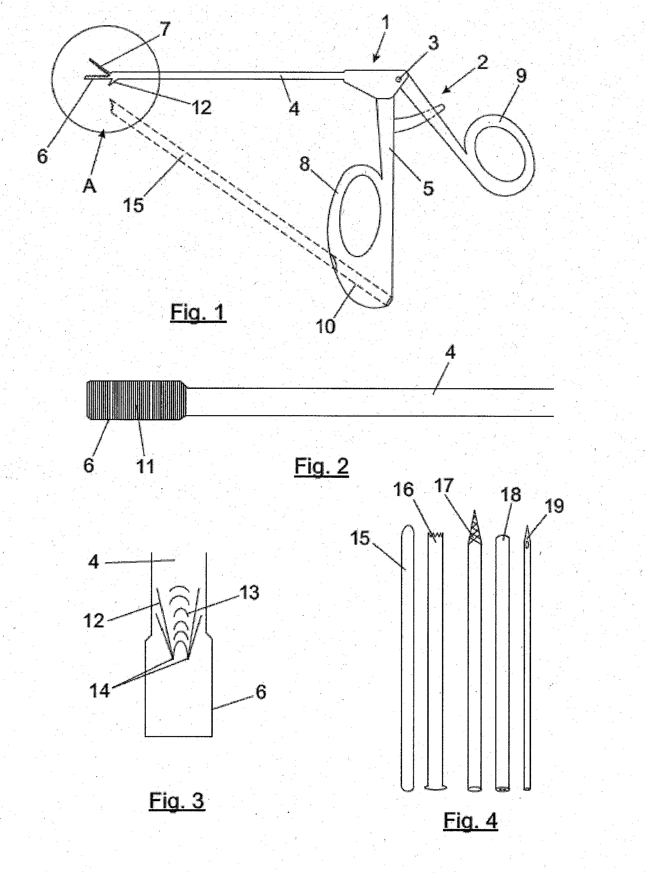

The forceps shown in figure 1, formed in accordance with the present invention, are formed by an elbow jointed arm, generally indicated by reference 1 and by a straight arm, 2, which is linked to the elbow jointed arm 1 by means of the articulation, 3, coinciding approximately with the elbow joint of the arm, 1.

The elbow jointed arm 1 comprises two stretches, 4 and 5, which form an angle of approximately 90° between them. Stretch 4 supports the jaws of the forceps at its free end, comprising one fixed jaw 6, which forms part of stretch 4 and another 7 being articulated to stretch 4 and linked by the same to the straight arm, 2, in order for it to operate.

The forceps are manipulated or handled using the operational hoops, 8 and 9, in which the two arms finish.

In accordance with the invention, the operational hoop 8 of the elbow jointed arm 1, has a drilled through hole 10, which is contained in the plane defined by stretches 4 and 5 of the elbow jointed arm and which slopes upwards towards stretch 4, sloping in such a way that it cuts said stretch immediately in front of the fixed jaw, 6, which is located at an approximate distance of 2mm away from the end of this jaw, 6.

Both the fixed jaw 6 and the mobile jaw 7 have a crosswise striation 11, as can be seen in figure 2.

According to another characteristic of the invention, stretch 4 of the elbow jointed arm 1 has a pointed outgoing 12 underneath the fixed jaw 6, immediately behind the same, which is forwards inclining. As can be better observed in figure 3, this outgoing has a striated surface 13 and its free end finishes in a double point or keel 14.

The outgoing 12 may incline at approximately 30°.

In order to carry a shoulder arthroscopy out, other known elements or instruments are used, which may comprise a blunt introducer 15 for a toothed sleeve 16, a spool 17, a blunt sleeve 18 and a needle 19, as shown in figure 4, the formation of all of these elements being known.

When using the forceps shown in figure 1 alongside the remaining traditional elements or instruments shown in figure 4, the steps to be followed in one embodiment of a shoulder arthroscopy would be as follows:
Step 1: Arthroscopic cleaning of the bursa of the subacromial shoulder area under direct vision, until the cuff and the tuberosity can be visualised correctly (this stage being common to any shoulder arthroscopy process).
Step 2: Introduction of the forceps, object of the present invention, through a cannula, before proceeding to take the torn tendon with the same, mobilising and carrying it to the point of the tuberosity at which the insertion of the same is to be made.
Step 3: Placing the forceps at the selected point, the keel 12 in the lower part of said forceps serving as a provisional bone mooring.
Step 4: With the tendon located at the selected point and the keel 12 acting as a bone mooring (placed laterally on the tuberosity), the toothed sleeve 16, as shown in figure 4, is introduced, with its blunt introducer 15, via a cutaneous incision of 0.5 cm. This sleeve will finally be supported on the cortical of the lateral face of the humerus, outside the tuberosity.
Step 5: The spool 17 is introduced via the toothed sleeve 16, which perforates the cortical and forms the osseous tunnel.
Step 6: The spool is extracted and is replaced by the blunt sleeve 18, which will be placed through to the output opening of the osseous tunnel, without removing the toothed sleeve.
Step 7: The needle 19 is introduced via the blunt sleeve 18 with the threaded thread, which will go through the tendon.
Step 8: The thread is recovered via a previously formed gateway before subsequently extracting the needle and both sleeves.
Step 9: The other tail end of the thread is recovered via the gateway.
Step 10: In this way, a thread has successfully been passed through an osseous tunnel and the torn tendon, in such a way that if a slipknot should be made, the tendon will have successfully been sutured against the tuberosity, a tendon surface measuring 15 mm pressing against the same.
Step 11: The thread is cut and the process can then be restarted or completed by means of a classic suture using moorings. In this way, both techniques can be used, in such a way that one complements the other or one may substitute the other in the event of technical complications in the first choice, depending on the patient, on the surgeons' expertise or on the type of suture required.

The drill hole 10 in the operational hoop 8 of the elbow jointed arm 1 serves as a positioner for the introduction of the blunt introducer 15 for the toothed sleeve 16 and the remaining elements described, thus making it possible to ensure its positioning.

Using the technology described, thanks to the forceps, object of the present invention, it is not necessary to fit moorings in order to suture the tendon, thus resulting in considerable savings. Furthermore, it is not necessary to fit foreign material in the patient's shoulder, thus eliminating the risk of a foreign body being rejected or producing a reaction.

Moreover, the forceps object of the present invention make it possible to carry out a simple technique which can be repeated, which successfully sutures the tendon in one single step. Consequently, this reduces time spent in surgery, thus producing benefits for the patient, reducing the risk of infection and reducing the need for anaesthetic. It is also beneficial to medical professionals and hospital centres, since it successfully optimises surgery time and enables the same resources to be used for a greater number of patients.

The technology described also facilitates contact between the bone and tendon measuring 15 mm, thus ending the eternal single/double thread debate and achieving earlier recovery by increasing the contact between the tendon and the bone. It also results in the patient being able to return to their everyday activities sooner.

Nevertheless, using the technique with the forceps, object of the invention, is not completely incompatible with employing classic techniques, it being possible to employ these or the same in more complex cases and/or in rescue surgery. The technique object of the present invention may even be used as a complementary technique to the same. **

In order to optimise the forceps, object of the present invention, the mouth of the forceps should be 13 mm deep, in order to ensure that 15 mm of tendon come into contact with the bone, when the needle perforates the tendon by 2 mm distal to the end of the forceps.

Moreover, the outgoing 12 should be approximately 4 mm in length, with an inclination of 30°, thus facilitating it being temporarily moored to the tuberosity and serving as a reference for subsequent mounting.

The stretch 5 of the elbow jointed arm 1 extends in order for the perforation 10 to enable the toothed guide with its blunt introducer 15, the spool 17, blunt sleeve 18 and needle 19 threaded with thread to pass, forming an angle such that it causes the spool to perforate the osseous tunnel, at a length and depth of 1 cm and at its output, approximately 2 mm coincides distal to the end of the toothed mouth of the forceps.

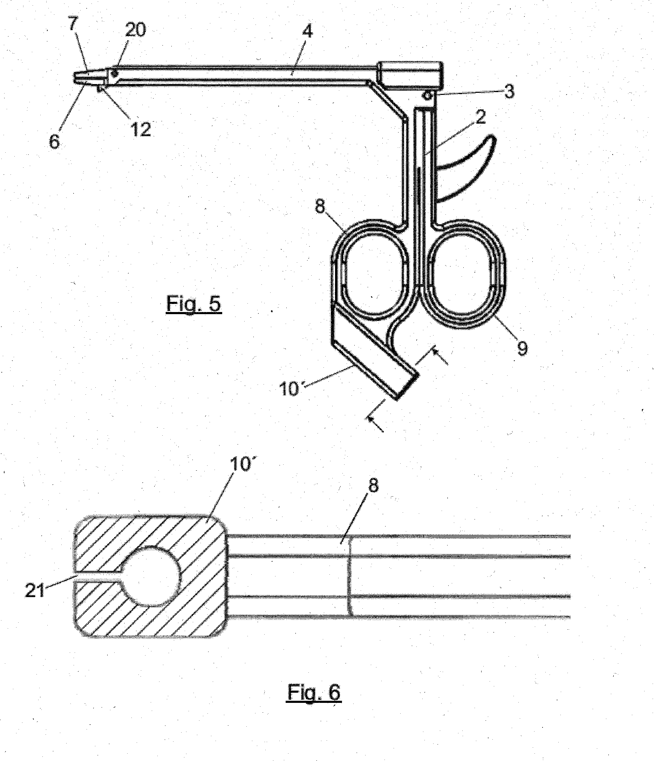

In the embodiment shown in figure 5, the articulation point 20 at which the clamp 7 is articulated to the fixed clamp 6 is represented. In this case, the drilled through hole of the operational hoop 8 is formed by tubing, 10' , which forms part of said ring and is opened longitudinally via a through opening 21, as shown in figure 6, which is wider than the diameter of the needle 19. The existence of this longitudinal opening facilitates the separation of the forceps once the needle 19 has been introduced into the osseous tunnel, via the tubing 10', without having to remove said needle.

## Claims

1. Shoulder arthroscopy forceps, comprising an elbow jointed arm 1 and a straight arm 2, the second being articulated in the elbow joint of the elbow jointed arm, this elbow jointed arm supporting the two jaws of the forceps at the end of one of its stretches 4, comprising one fixed 6 formed on the end of said stretch and another 7 articulated to the same, whilst the other stretch 5 and the straight arm 2 finish at the ends thereof in both operational hoops, 8 and 9, **characterized in that** the elbow jointed arm has a pointed outgoing, 12, on the supporting stretch of the fixed jaw of the forceps, behind and below said jaw, which is forwards inclining, whilst the operational ring 8 of the other stretch is crossed by a drilled through hole 10, located on the plane defined by said elbow jointed arm and running in an inclined position, in a trajectory which cuts said elbow jointed arm immediately in front of the end of the fixed jaw 6.

2. Forceps according to claim 1, **characterized in that** the abovementioned pointed outgoing 12 ends in a double point 14.

3. Forceps according to claim 1, **characterized in that** the abovementioned pointed outgoing has crosswise striation on the lower external surface 13.

4. Forceps according to claim 1, **characterised in that** the drill hole 10 which crosses the operational ring of the elbow jointed arm has dimensions which facilitate the passing of the toothed guide with its blunt introducer 15 through the same, as well as the remaining necessary instruments.

5. Forceps according to claim 1, **characterised in that** the drill hole which crosses the operational ring 8 forms tubing 10', which forms part of said ring and is opened longitudinally via a through opening, 21.
